# EUROPEAN PATENT APPLICATION

(11) **EP 2 520 581 A1**
(43) Date of publication of application: **07.11.2012**
(21) Application number: 12178644.6
(22) Date of filing: 06.08.2009
(51) Int. Cl.: C07H 19/12, C07H 1/00, A61K 31/706

(54) **Method for the determination the purity of 5-Azacytidine**

(30) Priority: 06.08.2008 US 86606 P; 21.01.2009 US 146112 P; 14.05.2009 US 178309 P
(62) Divisional of application: 09791228.1
(71) Applicant: Sicor, Inc., Irvine, CA 92618 (US)
(72) Inventor: Bigatti, Ettore, I-20017 Rho (IT); Lux, Giovanna, I-20131 Milano (IT); Paiocchi, Maurizio, I-20152 Milano (IT); Giolito, Andrea, I-20090 Vimodrone MI (IT); Tosi, Simone, I-20014 Nerviano MI (IT)
(74) Representative: Gallagher, Kirk James

(57) **Abstract**

The present invention further provides an analytical method for determining the purity of 5-Azacytidine in a sample, said method comprising :

a) providing a sample of 5-Azacytidine,

b) dissolving a first part of the sample in dimethyl sulfoxide ("DMSO"), and a second part of the sample in dimethylpropyleneurea ("DMPU"), providing a first and a second sample solution,

c) injecting each sample solution onto an HPLC column under the same conditions, providing a first and a second chromatogram, and

d) determining the purity of 5-Azacytidine and the amount of each impurity based on both chromatograms.

## Description

### CROSS REFERENCE TO RELATED APPLICATIONS

The present invention claims the benefit of the following United States Provisional Patent Application Nos.: 61/086,606, filed August 6, 2008; 61/146,112, filed January 21, 2009; and 61/178,309, filed May 14, 2009. The contents of these applications are incorporated herein by reference.

### Field of the Invention

The invention encompasses a process for preparing an intermediate of 4-amino-1-β-D-ribofuranosyl-1, 3, 5-triazin-2 (1H)-one (5-Azacytidine), 4-Amino-1-(2, 3,5-tri-ester-β-D-ribosyl)-s-triazin-2(1H)-one, using protic acid as a catalyst.

### Background of the Invention

5-Azacytidine, 4-amino-1-β-D-ribofuranosyl-1, 3, 5-triazin-2(1H)-one, a compound having the chemical structure, 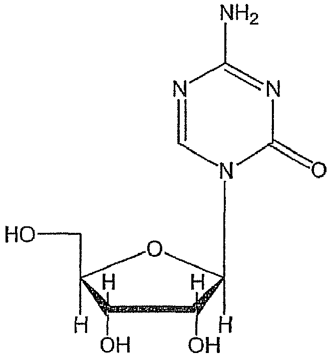 is an antineoplastic drug exhibiting activity against, e.g., leukemia, lymphoma and various solid tumours. 5-Azacytidine acts also as an inhibitor of DNA methyltransferase and was approved for the treatment of myelodysplastic syndromes, a family of bone-marrow disorders. It is being marketed under the name VIDAZA® by Pharmion.

The preparation of 5-Azacytidine by coupling silylated 5-azacytosine with sugar moiety is reported in US patent No. 3,817,980 and in US patent No. 7,038,038.

The process can be illustrated by the following scheme. 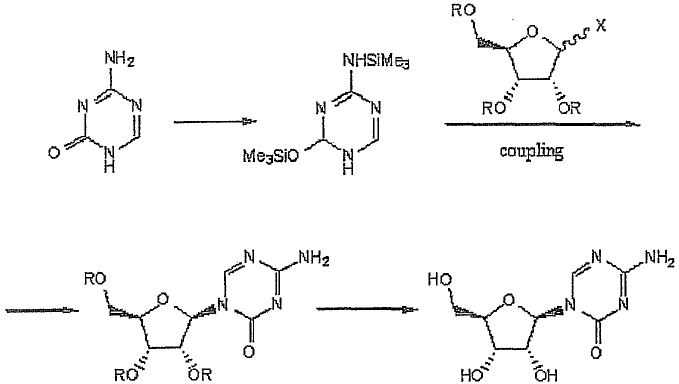 wherein, in US patent No. 3,817,980 R is benzoyl and X is O-acetyl, and the coupling process is done by using metallic Lewis acids, such as SnCl₄, TiCl₄, ZnCl₂, as catalysts (The reported yield of this process is 54.1 %.); in US patent No. 7,038,038 R is acetyl and X is O-acetyl, and the coupling process is done by using non-metallic Lewis acids as catalysts. (The reported yield of this process is 44.9%.); and in M. W. Winkley and R K. Robins, J. Org. Chem., 35, 491 (1970), X is Br, R is acetyl and the process is done without Lewis acid (The reported yield of this process is 34%.).

The use of metallic Lewis acids is known to cause contamination of the final product with traces of the metal that are formed, as reported in US patent No. 7,038,038. Also, all processes result in low yields of the final, 5-Azacytidine.

The invention described herein refers to an improved process for the preparation of 5-Azacytidine in higher yield, via its intermediate, 4-amino-1-(2, 3, 5-triester-β-D-ribosyl)-s-triazin-2(1H)-one, which is prepared by coupling of silylated 5-azacytosine with halide- sugar moiety in the presence of a protic acid instead of Lewis acids.

### Summary of the invention

In one embodiment, the present invention encompasses a process for preparing an intermediate of 4-amino-1-β-D-ribofuranosyl-1, 3, 5-triazin-2(1H)-one ("5-Azacytidine"), 4-amino-1-(2, 3, 5-tri-ester-β-D-ribosyl)-s-triazin-2(1H)-one, of the formula I: 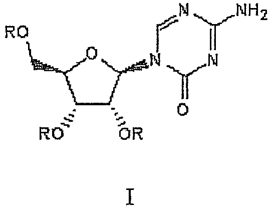 comprising reacting a silylated 5-azacytosine of the formula II, 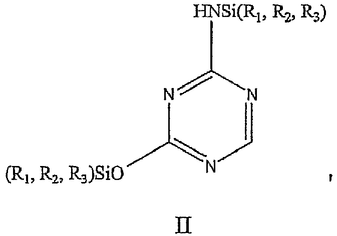 a sugar moiety having of the formula III: 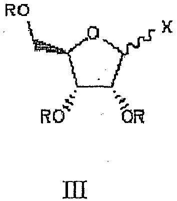 and a protic acid; wherein R is a substituted or non substituted C₁-C₂₀ acyl moiety R₁, R₂ and R₃ are each independently H or an alkyl group, and X is a halogen

In another embodiment, the present invention encompasses a process for preparing 4-amino-1-β-D-ribofuranosyl-1, 3, 5-triazin-2(1H)-one ("5-Azacytidine") of the formula IV: 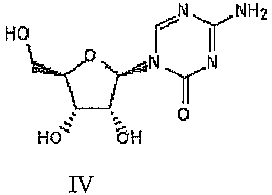 comprising preparing the intermediate of 5-Azacytidine of the formula I according to the process of the present invention, and converting it to 4-amino-1-β-D-ribofuranosyl-1, 3, 5-triazin-2(1H)-one ("5-Azacytidine").

In one embodiment, the present invention provides a method for determining the purity of 5-Azacytidine comprising:
a) providing a sample of 5-Azacytidine,
b) dissolving a first part of the sample in dimethyl sulfoxide ("DMSO"), and a second part of the sample in dimethylpropyleneurea ("DMPU"), providing a first and a second sample solution,
c) injecting each sample solution onto an HPLC column under the same conditions, providing a first and a second chromatogram, and
d) determining the purity of 5-Azacytidine and the amount of each impurity based on both chromatograms.

### Brief description of the figures

Figure 1 shows a PXRD pattern of 4-Amino-1-(2, 3, 5-tri-*O*-acetyl-β-D-ribosyl)-s-triazin-2(1H)-one.

Figure 2 shows a DSC thermogram of 4-Amino-1-(2, 3, 5-tri-*O*-acelyl-β-D-ribosyl)-s-triazin-2(1H)-one.

Figure 3 shows a PXRD pattern of 2-(Trimethylsilylamino)-4-(trimethylsilyloxy)-s-triazine.

Figure 4 shows a DSC thermogram of 2-(Trimethylsilylamino)-4-(trimethylsilyloxy)-s-triazine.

Figure 5 shows a PXRD pattern of 5-azacytosine

Figure 6 shows a FTIR spectrum of 5-azacytosine.

Figure 7 shows a HPLC chromatogram of 5-Azacytidine dissolved in DMSO.

Figure 8 shows a HPLC chromatogram of 5-Azacytidine dissolved in DMPU.

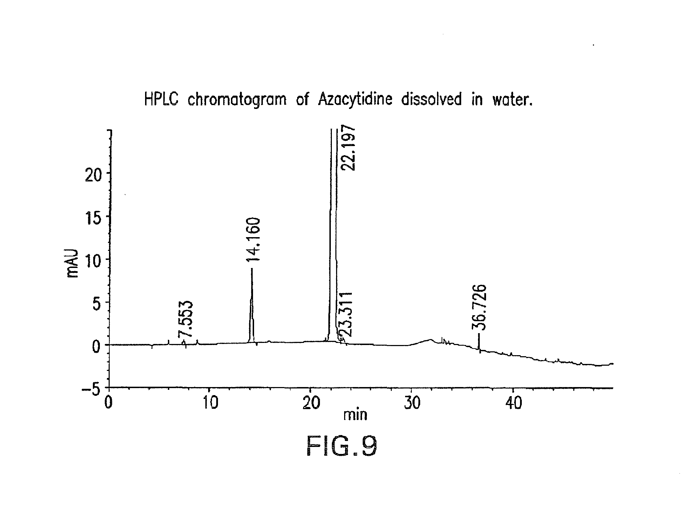

Figure 9 shows a HPLC chromatogram of 5-Azacytidine dissolved in water.

### Detailed description of the invention

The present invention relates to an improved process for the preparation of 5-Azacytidine in higher yield, via its intermediate, 4-amino-1-(3, 5-tri-ester-β-D-ribosyl)-s-triazin-2(1H)-one, and a method to determine its purity.

As used herein in connection with a measured quantity, the term "about" refers to that variation in the measured quantity as would be expected by the skilled artisan performing the measurement and exercising a level of care commensurate with the objective of the measurement and the precision of the measuring apparatus being used.

As used herein the term "purity" and "pure" relate to the chemical purity of a compound which may contain other chemical compounds as impurities wherein the particular compound is present in an amount of at least about 80%, preferably at least about 95%, more preferably at least about 99%, most preferably at least about 99.5% by weight. Typically, the purity can be measured by HPLC, for example by the HPLC method provided by the present invention.

As used herein the term "Acyl" refers to a radical having the general formula R" C(O) -, where R" is hydrogen, alkyl, cycloalkyl, cycloheteroalkyl, aryl, aralkyl, heteroalkyl, heteroaryl, heteroarylalkyl.

As used herein the term "Aralkyl" alone or as part of another substituent refers to a radical in which an aryl group is substituted onto an alkyl group radical. Typical aralkyl groups include, but are not limited to, benzyl, 2-phenylethan-1-yl, 2-phenylethen-1-yl, naphthylm ethyl, 2-naphthylethan-1-yl, 2-naphthylethen-1-yl, naphthobenzyl, 2-naphthophenylethan-1-yl and the like.

The process of the present invention applies a protic acid in the coupling step, instead of metallic or non-metallic Lewis acids. The coupling reaction mixture can be used in the next step without removing the acid. If desired, the protic acid can be removed by extraction with a base as compared to the difficulty in removing the metallic Lewis acids from the final product. Also, protic acids are comparatively cheaper compared to metallic and non metallic Lewis acids, thus resulting in a cost effective process that can also be applied in large-scale.

The process can be illustrated by the following scheme:

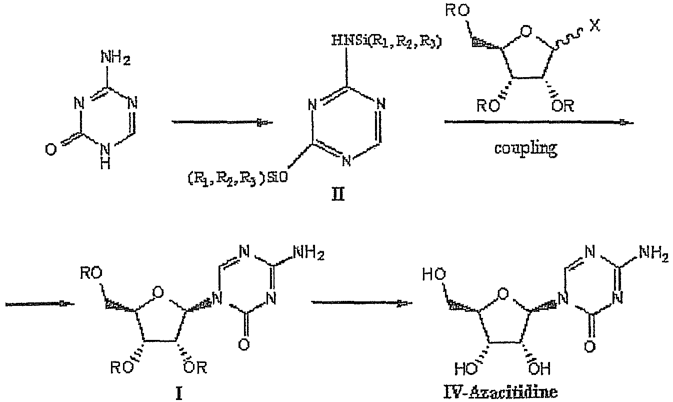

wherein R is a substituted or non substituted C₁-C₂₀ acyl moiety, R₁, R₂ and R₃ are independently H or an alkyl group, and X is a halogen.

The preparation of 5-Azacytidine intermediate of formula I comprises reacting silylated 5-azacytosine of the formula II 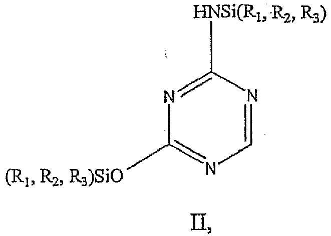 a sugar moiety of the formula III 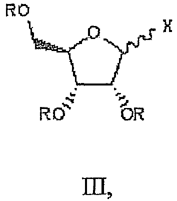 and a protic acid, wherein R is a substituted or non substituted C₁-C₂₀ acyl moiety, R₁, R₂ and R₃ are each independently H or an alkyl group, and X is a halogen..

Preferably, the C₁-C₂₀ acyl moiety is substituted with an aliphatic or branched alkyl, or with a benzyl group.

Preferably, the C₁-C₂₀ acyl moiety is C (O) CH₃ or C (O) phenyl (i.e. R is C (O) CH₃ or C (O) phenyl), most preferably, C (O) CH₃.

Preferably, the alkyl group in the R₁, R₂ and R₃ of formula II is a C₁-C₄ alkyl group, more preferably, C₁-C₂ alkyl, most preferably, R₁= R₂ = R₃= methyl.

Preferably, the halogen is either Cl or Br.

When R is C (O) CH₃, the compound of formula I corresponds to 4-Amino-1-(2, 3, 5-tri-*O*-acetyl-β-D-ribosyl)-s-triazin-2(1H)-one, having the following formula,

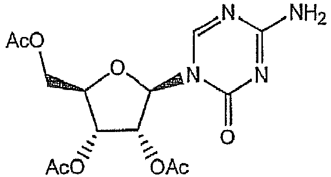

Preferably, the obtained 4-Amino-1-(2, 3, 5-tri-*O*-acetyl-β-D-ribosyl)-s-triazin-2(1H)-one of the above formula corresponding to formula I is crystalline.

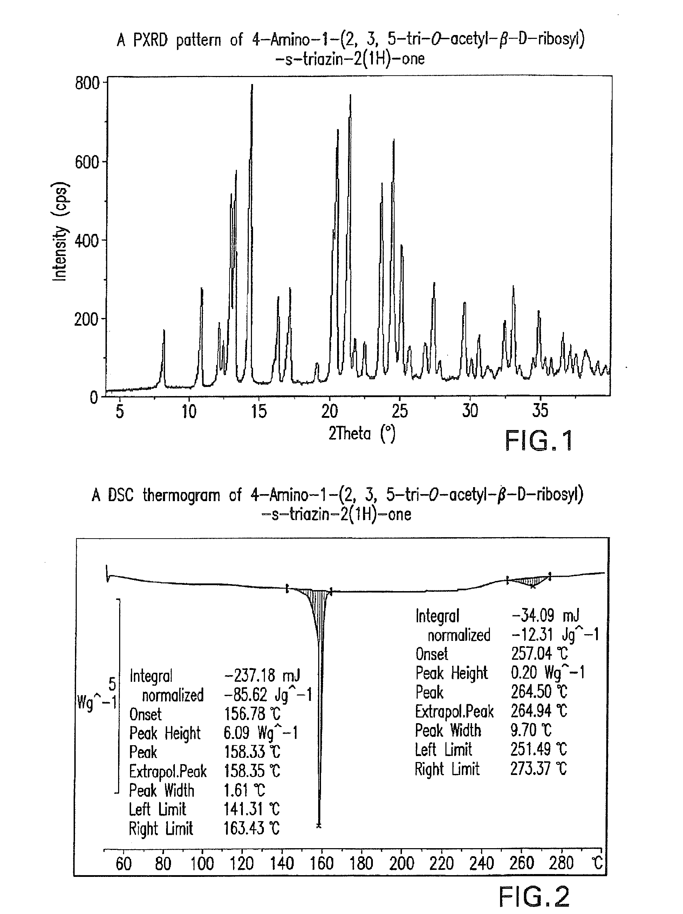

The crystalline 4-Amino-1-(2,3,5-tri-*O*-acetyl-β-D-ribosyl)-s-triazin-2(1H)-one, is characterized by data selected from the group consisting of: a PXRD pattern having peaks at about 8.2, 10.9, 13.0, 13.3, 14.3, 16.4, 17.2, 20.4, 21.3, 23.7, 24.4, 25.1 and 27.4 ± 0.2 deg. 2θ, and a PXRD pattern as depicted in figure 1.

The crystalline 4-Amino-1-(2,3,5-tri-*O*-acetyl-β-D-ribosyl)-s-triazin-2(1H)-one, maybe further characterized by data selected from the group consisting of: a DSC thermogram having an Endothermic peak at about 158°C, and a DSC thermogram as depicted in Figure 2.

When R₁= R₂ = R₃= methyl, the compound of formula II corresponds to 2-(Trimethylsilylamino)-4-(trimethylsilyloxy)-s-triazine of the following formula,

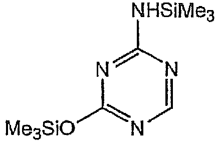

Preferably, the 2-(Trimethylsilylamino)-4-(trimethylsilyloxy)-s-triazine, is crystalline.

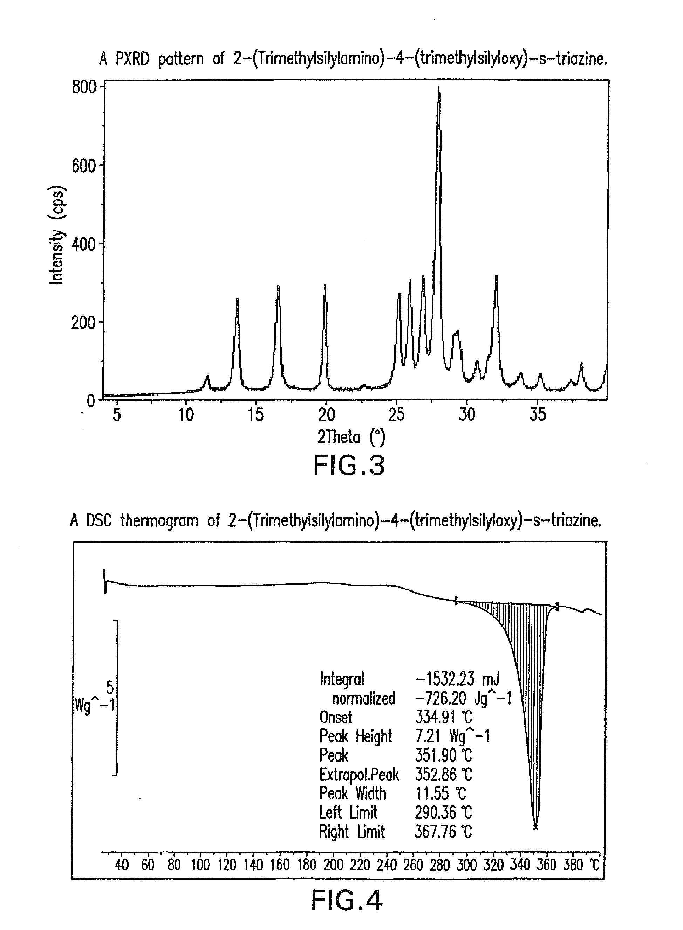

The crystalline 2-(Trimethylsilylamino)-4-(trimethylsilyloxy)-s-triazine is characterized by data selected from the group consisting of: a PXRD pattern having peaks at about 11.6, 13.7, 16.6, 19.9, 25.2, 26.0, 26.9, 27.8, 29.1, 30.7, 32.1, 35.2 and 38.2 ± 0.2 deg. 2θ, and a PXRD pattern as depicted at figure 3.

The crystalline 2-(Trimethylsilylamino)-4-(trimethylsilyloxy)-s-triazine can be further characterized by data selected from the group consisting of: a DSC thermogram having an Endothermic peak at about 352°C, and a DSC thermogram as depicted in figure 4.

In a preferred embodiment, the present invention encompasses the preparation of the 5-Azacytidine intermediate of formula I by combining a first mixture comprising the silylated 5-azacytosine of formula II, a second mixture comprising the sugar moiety of formula III and a protic acid to obtain a reaction mixture, comprising said intermediate of formula I.

In some embodiment, the protic acid is present in a catalytic amount, preferably, the protic acid is present in an amount of about 0.1 to about 0.9 mol/mol, in respect to the silylated 5-Azacytosine of formula II.

Preferably, the protic acid in the processes of the invention is triflic acid.

The first mixture comprises the silylated 5-azacytosine of formula II and an organic solvent. Examples for suitable organic solvents include but are not limited to acetonitrile, methylene chloride and 1, 2- dichloromethane, preferably, the organic solvent is acetonitrile.

The preparation of the silylated 5-azacytosine of formula II comprises the use of an organic solvent instead of using the expensive silylating agent also as a solvent. Thus, only a stoichiometric to small excess of the silylating agent, which is expensive, is used.

Preferably the silylating agent has the following formula (R₁R₂R₃) Si-NH-Si (R₁R₂R₃), wherein R₁, R₂ and R₃ are independently H or C₁-C₄ alkyl. More preferably, the silylating agent is hexamethyldisilazane (HMDS).

The first mixture is provided by combining 5-azacytosine having the following formula, 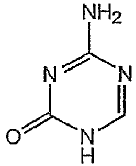 with the silylating agent and a solvent to obtain a first suspension; heating the first suspension to obtain a solution, evaporating the solution to obtain a residue, and combining the residue and the organic solvent, preferably acetonitrile, to obtain said mixture comprising silylated 5-azacytosine of formula II.

Preferably, the starting 5-azacytosine is crystalline.

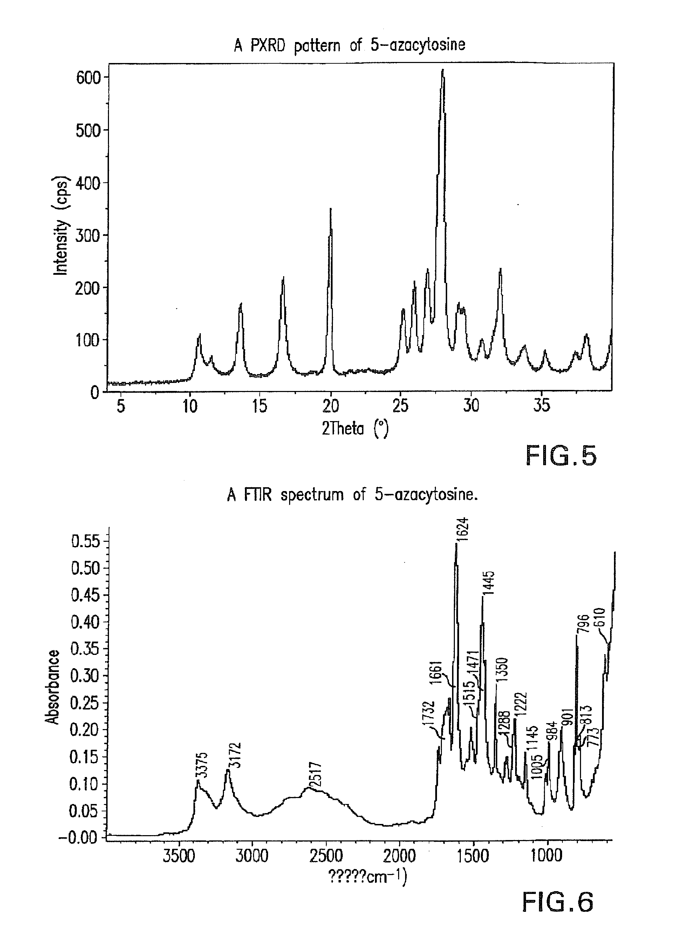

The crystalline 5-azacytosine is characterized by data selected from the group consisting of: a PXRD pattern having peaks at about 11.6, 13.7, 16.6, 19.9, 25.2, 26.0, 26.9, 27.8, 29.1, 30.7, 32.1, 35.2 and 38.2 ± 0.2 deg. 2θ, and a PXRD pattern as depicted in figure 5.

The crystalline of 5-azacytosine maybe further characterized by data selected from the group consisting of: a FTIR spectrum having bands at about 3375, 3172, 2617, 1732, 1661, 1624, 1515, 1471, 1445, 1350, 1269, 1222, 1145, 1006, 984, 901, 813, 796, 773 and 610 cm⁻¹, and a FTIR spectrum as depicted in figure 6.

Examples for suitable organic solvents used to prepare the silylated 5-azacytosine of formula II include but are not limited to an aromatic hydrocarbon, preferably a C₆₋₉ aromatic hydrocarbon, more preferably toluene.

The silylating agent is present between stoichiometric amount to small access per the amount of 5-azacytosine. Preferably, about 1.0 to about 2.0 mol, more preferably, about 1.4 to about 1.6 mol equivalent of the silylating agent per mol equivalent of 5-azacytosine, is reacted.

Optionally, the first mixture comprises also a catalyst such as (NH₄)₂SO₄.

The first suspension is heated to obtain a solution. Preferably, the first suspension is heated to a temperature of about 80°C to about 120°C. More preferably, the first suspension is heated to a temperature of about 104°C to about 120°C.

Preferably, the first suspension is heated for about 1 to about 6 hours, more preferably, for about 4 hours.

Optionally, the solution can be further heated to ensure the formation of the silylated 5-azacytosine. Preferably, the solution can be further heated for about 1 to about 6 hours, more preferably, for about 4 hours.

As mentioned above the solution is evaporated to give a residue.

The evaporation process can be repeated several times, prior to combining the residue with the organic solvent. Preferably, the organic solvent is acetonitrile.

Usually, prior to each evaporation step the obtained residue is combined with a solvent to obtain a solution and this solution is evaporated. Preferably, the solvent is an aromatic hydrocarbon, more preferably a C₆₋₉ aromatic hydrocarbon, most preferably toluene.

When X is Cl and R is C (O) CH₃, the sugar moiety of formula III is 2, 3, 5-tri-O-acetyl-ribofuranosyl chloride, and can be prepared for example according to the process described in A. Piskala and F. Storm, Nucl. Acid Chem. 1, 435 (1978), incorporated herein by reference in its entirety.

The second mixture can be a solution of the sugar moiety of formula III in an organic solvent. Examples for suitable organic solvents include but are not limited to acetonitrile, methylene chloride and 1, 2- dichloromethane, preferably, the organic solvent is acetonitrile.

Further, the reaction mixture comprising all reactants, after combining the first and second mixtures and a protic acid, is stirred preferably for a period of about 6 to 30 hours, more preferably for about 20 to 26 hours, most preferably for about 22 to 24 hours allowing the formation of the intermediate 4-amino-1-(2, 3, 5-tri-ester-β-D-ribosyl)-s-triazin-2(1H)-one of formula I.

Preferably, the stirring is performed at a temperature of about 20°C to about 30°C, more preferably, at a temperature of about 23°C to about 27°C, most preferably, at a temperature of about 24°C to about 26°C.

Prior to converting the obtained intermediate 4-amino-1-(2, 3, 5-tri-ester-β-D-ribosyl)-s-triazin-2(1H)-one of formula I to 5-Azacytidine the reaction mixture containing it is concentrated to obtain a residue comprising the intermediate 4-amino-1-(2, 3, 5-tri-ester-β-D-ribosyl)-s-triazin-2(1H)-one of formula I, which reacts with a base to neutralize the protic acid.

Optionally, prior to reacting the residue with the base, it is dissolved in a water-immiscible organic solvent, providing a solution which then reacts with an aqueous solution of the base. Examples for suitable water-immiscible organic solvents include but are not limited to a halogenated aliphatic hydrocarbon or ester, preferably, the halogenated aliphatic hydrocarbon is a C₁₋₃ halogenated aliphatic hydrocarbon and the ester is a C₁₋₆ ester. More preferably, the C₁₋₃ halogenated aliphatic hydrocarbon is CH₂Cl₂ or CHCl₃, and the C₁₋₆ ester is AcOEt.

Preferably, the base is an inorganic base. Examples for suitable inorganic base include but are not limited to NaHCO₃, Na₂CO₃, K₂CO₃, KHCO₃, NaOH and NH₄OH. More preferably, the base is sodium bicarbonate

Ordinarily, the reaction with the base provides a mixture. This mixture is filtered providing a filtrate, which is then concentrated to give an oil comprising the intermediate 4-amino-1-(2, 3, 5-tri-ester-β-D-ribosyl)-s-triazin-2(1H)-one of formula I.

The obtained intermediate of formula I can then be converted to 5-Azacytidine. Typically, the conversion is done by removing the acetylated protecting groups. The removal can be done by reacting the intermediate of formula I with a base, for example as reported herein in example 1 or by the procedure described in US patent No. 7,038,038. The yield of 5-azacytidine according to the process of the invention is at least 65%, preferably at least 69%.

Typically, the purity of the obtained 5-Azacytidine is then analyzed. However, since Azacytidine is very unstable in water and has a low solubility in common solvents used in HPLC analysis, a different method of analysis is required.

The current invention provides such different analytical method using HPLC to determine the purity of 5-Azacytidine. The method for determining the percentage purity by area HPLC of 5-Azacytidine is provided, comprising:
a) providing a sample of 5-Azacytidine,
b) dissolving a first part of the sample in DMSO, and a second part of the sample in DMPU, providing a first and second sample solution,
c) injecting each sample solution onto an HPLC column under the same conditions, providing a first and second chromatogram, and
d) determining the purity of 5-Azacytidine and the amount of each impurity based on both chromatograms.

Preferably, the concentration of each sample solution in step b) is determined prior to the injection onto the HPLC column. More preferably, the concentration of each sample solution in step b) is of about 2.7 mg/ml to about 3.3 mg/ml, most preferably about 3mg/ml.

Typically, step d is done by subtracting the total percentage of impurities from 100%. Total percentage of impurities is obtained as the sum of the percentage of each impurity (having relative retention time not less than 0.5) detected in the sample dissolved in DMSO and the percentage of each impurity (having relative retention time less than 0.5) detected in the sample dissolved in DMPU.

If the purity is not sufficient, 5-Azacytidine can be further purified, for example by crystallization. The crystallization can comprise providing a suspension of 5-Azacytidine in DMPU to obtain a mixture and filtering said mixture to obtain a solid. The obtained solid is then suspended in isopropyl alcohol. 5-azacytidine is then obtained by drying said suspension.

Having described the invention with reference to certain preferred embodiments, other embodiments will become apparent to one skilled in the art from consideration of the specification. The disclosures of the references referred to in this patent application are incorporated herein by reference. The invention is further defined by reference to the following examples describing in detail the process and compositions of the invention. It will be apparent to those skilled in the art that many modifications, both to materials and methods, may be practiced without departing from the scope of the invention

### EXAMPLES

### HPLC method for monitoring the purity of Azacytidine:

Column: Reversed Phase silica C18 (octadecyl; 5 µm, 250 x 4.6 mm or equivalent,

Mobile Phase A: 15mM of Potassium Phosphate Dibasic and 15mM of Ammonium Formate in Water, adjust with diluted Orthophosphoric Acid (10 mL of 85% orthophosphoric Acid to 100 mL of Water) to pH 7.0 ± 0.1.

Mobile Phase B: Phase A/Acetonitrile 60:40 (v/v)

Gradient:

| **Time (min)** | **Mobile Phase A (%)** | **Mobile Phase B (%)** |
|---|---|---|
| 0 | 100 | 0 |
| 30 | 95 | 5 |
| 50 | 55 | 45 |
| 55 | 50 | 50 |
| 80 | 50 | 50 |

Run Time: 50minutes.

Post Run Time: 15 minutes.

Flow Rate: 0.7 mL/min.

Detector: λ = 235 nm (ref. = 450nm, BW=80nm).

Column Temperature: 15 °C.

Injection Volume: 2 µL.

Diluent A: Dimethylsulfoxide (DMSO)

Diluent B: 1, 3-dimethyl-3, 4, 5, 6-tetrahydro-2(1H)-pyrimidinone (DMPU)

Autosampler Temperature:Autosampler temperature must be maintained above 20°C to prevent DMSO freezing when it is used as solvent.

### PXRD

XRD diffraction was performed on X-Ray powder diffractometer: PanAlytical X'pert Pro powder diffractometer equipped with X'celerator multichannel detector, detector active length 2.122 mm, Cu-tube, CuKα radiation, λ = 1.541874 Å; a stainless steel sample holder with zero background silicon plate. Scanning parameters: Range 4-40 degrees two-theta; Continuous scan; Step size 0.0167 deg; Scan rate 6 deg./min._Prior to analysis the samples were gently ground by means of mortar and pestle in order to obtain a fine powder. The ground sample was adjusted into a cavity of the sample holder and the surface of the sample was smoothed by means of a microscopic glass slide.

### DSC

DSC measurements were performed on Differential Scanning Calorimeter DSC823e (Mettler Toledo). Al crucibles 40 µl with PIN were used for sample preparation. Usual weight of sample was 1.5 ― 4 mg.
Program: temperature range 25°C - 300°C, 10°C/min, Nitrogen flow 50ml/min.
IR

FTIR spectra were collected by means of a spectrometer Nicolet Nexus. ATR technique was used for the measurement with the following settings:

| | |
|---|---|
| Range: | 4000 - 550 cm⁻¹; |

| | |
|---|---|
| Number of sample scans: | 64; |
| Resolution: | 4.000 cm⁻¹; |
| Apodization: | Happ-Genzel; |
| Sample gain: | 8.0; |
| Final format: | Absorbance. |

The empty ATR crystal was measured as a background under the same conditions as were the samples. The resulting record was then subtracted automatically from the spectra of the samples.

The above-mentioned experimental methods were used to measure the various parameters in the Examples below.

### Example 1: Synthesis of 5-Azacytidine

A suspension of 5-azacytosine (10 g; 0.089 mol), hexamethyldisilazane (22 g; 0.13 mol), (NH₄)₂SO₄ (0.2 g; 2 mmol) and toluene (40 g) was heated to reflux (Tₑₓₜ 130°C; Tmix 108-114°C). After about 4 h the mixture became a solution and the reaction was refluxed for additional 4 h. The solution was evaporated under vacuum to oil, which was diluted with toluene (50 g) and the resulting solution was evaporated under vacuum to residue. The latter was suspended in CH₃CN (60 g) obtaining a suspension (Mixture A).

β-D-ribofuranose tetracetate (34.4 g; 0.11 mol) was suspended in toluene (150 g) and acetyl chloride (1.7 g; 0.02 mol) was added. The mixture was stirred at 20-25°C and HCl gas (5.46 g; 0.15 mol) was bubbled over about 8 h (IPC by TLC Eluent: CH₂Cl₂/acetone 95:5; Residual SM<5%).

The reaction mixture (solution) was evaporated under vacuum (external bath 50°C) to an oily residue, which was then dissolved in CH₃CN (102 g) obtaining a solution (Mixture B).

The solution of 2, 3, 5-tri-*O*-acetyl-D-ribofuranosyl chloride in CH₃CN (Mixture B) was poured into the suspension of bis-trimethylsilyl-azacytosine in CH₃CN (Mixture A) over 10 min and triflic acid (5.4 g; 0.04 mol) was added.

The resulting mixture was stirred at 20-25°C for 20-25 h, then it was concentrated to residue. The latter was dissolved in CH₂Cl₂ (200 g) and the resulting solution was treated with a mixture of NaHCO₃ (7.5 g; 0.09 mol) and Na₂CO₃ (9.5 g; 0.09 mol) in H₂O (100 g) and vigorously stirred for 30 min.

The mixture was filtered on a dicalite cake, then the phases were separated and the aqueous layer was extracted with CH₂Cl₂ (30 g).

The organic phases were collected and concentrated under vacuum (50°C) to an oily residue.

The latter was dissolved in MeOH (250 g) and the resulting mixture was filtered (small amount of salt).

25% MeONa/MeOH (3.9 g; 0.02 mol) dissolved in MeOH (60 g) was added over 30 min.

The resulting mixture is stirred at 20-25°C for 1.5 h. The crystals were filtered and washed with MeOH (300 g).

The wet solid was dried under vacuum at 60°C for 15 h to give 5-azacytidine as an off white solid.

Yield: 69% purity> 98% area by HPLC.

### Example 2: Synthesis of 5-Azacytidine

A suspension of 5-azacytosine (10 g; 0.089 mol), hexamethyldisilazane (22 g; 0.13 mol), (NH₄)₂SO₄ (0.2 g; 2 mmol) and toluene (40 g) was heated to reflux (Tₑₓₜ 130°C; Tmix 108-114°C). After about 4 h the mixture became a solution and the reaction was refluxed for additional 4 h. The solution was evaporated under vacuum to oil, which was diluted with toluene (50 g) and the resulting solution was evaporated under vacuum to residue. The latter was suspended in CH₃CN (60 g) obtaining a suspension (Mixture A).

β-D-ribofuranose tetracetate (34.4 g; 0.11 mol) was suspended in toluene (150 g) and acetyl chloride (1.7 g; 0.02 mol) was added. The mixture was stirred at 20-25°C and HCl gas (5.46 g; 0.15 mol) was bubbled over about 8 h (IPC by TLC Eluent: CH₂Cl₂/acetone 95:5; Residual SM<5%).

The reaction mixture (solution) was evaporated under vacuum (external bath 50°C) to an oily residue, which was then dissolved in CH₃CN (102 g) obtaining a solution (Mixture B).

The solution of 2, 3, 5-tri-*O*-acetyl-D-ribofuranosyl chloride in CH₃CN (Mixture B) was poured into the suspension of bis-trimethylsilyl-azacytosine in CH₃CN (Mixture A) over 10 min and triflic acid (5.4 g; 0.04 mol) was added.

The resulting mixture was stirred at 20-25°C for 20-25 h, and then it was concentrated to residue. The latter was dissolved in CH₂Cl₂ (200 g) and the resulting solution was treated with a mixture of NaHCO₃ (7.5 g; 0.09 mol) and Na₂CO₃ (9.5 g; 0.09 mol) in H₂O (100 g) and vigorously stirred for 30 min.

The mixture was filtered on a dicalite cake, then the phases were separated and the aqueous layer was extracted with CH₂Cl₂ (30 g).

The organic phases were collected and concentrated under vacuum (50°C) to an oily residue.

The latter was dissolved in MeOH (240 g) and the resulting mixture was filtered (small amount of salt).

25% MeONa/MeOH (15.4 g; 0.07 mol) dissolved in MeOH (250 g) was added over 5 min.

The resulting mixture is stirred at 20-25°C for 1.5 h. The crystals were filtered and washed with MeOH (300 g).

The wet solid was dried under vacuum at 60°C for 15 h to give 5-azacytidine as an off white solid.

Yield: 69%,, purity >99% area by HPLC.

### Example 3: Synthesis of 5-Azacytidine without aqueous basic work-up

A suspension of 5-azacytosine (10 g; 0.089 mol), hexamethyldisilazane (22 g; 0.13 mol), (NH₄)₂SO₄ (0.2 g; 2 mmol) and toluene (40 g) was heated to reflux (Tₑₓₜ 130°C; Tₘᵢₓ 108-114°C). After about 4 h the mixture became a solution and the reaction was refluxed for additional 4 h. The solution was evaporated under vacuum to oil, which was diluted with toluene (50 g) and the resulting solution was evaporated under vacuum to residue. The latter was suspended in CH₃CN (60 g) obtaining a suspension (Mixture A).

β-D-ribofuranose tetracetate (34.4 g; 0.11 mol) was suspended in toluene (150 g) and acetyl chloride (1.7 g; 0.02 mol) was added. The mixture was stirred at 20-25°C and HCl gas (5.46 g; 0.15 mol) was bubbled over about 8 h (IPC by TLC Eluent: CH₂Cl₂/acetone 95:5; Residual SM<5%).

The reaction mixture (solution) was evaporated under vacuum (external bath 50°C) to an oily residue, which was then dissolved in CH₃CN (102 g) obtaining a solution (Mixture B).

The solution of 2, 3, 5-tri-*O*-acetyl-D-ribofuranosyl chloride in CH₃CN (Mixture B) was poured into the suspension of bis-trimethylsilyl-azacytosine in CH₃CN (Mixture A) over 10 min and triflic acid (5.4 g; 0.04 mol) was added.

The resulting mixture was stirred at 20-25°C for 20-25 h, and then it was concentrated to residue.

The latter was dissolved in MeOH (240 g) and the resulting mixture was filtered (small amount of salt).

25% MeONa/MeOH (15.4 g; 0.02 mol) dissolved in MeOH (250 g) was added over 5 min.

The resulting mixture is stirred at 20-25°C for 1.5 h. The crystals were filtered and washed with MeOH (300 g).

The wet solid was dried under vacuum at 60°C for 15 h to give 5-azacytidine as an off white solid.

Yield: 75%, purity >99% area by HPLC.

### Example 4: Preparation of crystalline 4-amino-1, 2-dihydro-1, 3, 5-triazin-2-one:

A mixture of guanylurea (1.02 g; 0.01 mol), DMF (5 mL) and ethyl ortoformate (3mL) was heated at 155°C for 1.5 h and then allowed to stand at room temperature overnight. The suspension was filtered and the solid washed with H₂O (5 mL). 5-Azacytosine was re-crystallized from H₂O.

### Examples 5: Preparation of crystalline 2-(Trimethylsilylamino)-4-(trimethylsilyloxy)-s-triazine:

A mixture of 5-azacytosine (11.2 g; 0.1 mol), hexamethyldisilazane (35 mL) and ammonium sulphate (0.2 g) was heated to reflux (oil bath 160°C) for 8 h. The excess of hexamethyldisilazane was evaporated under vacuum obtaining a residue which was triturated with dry toluene (50 mL) and the solvent was evaporated under reduced pressure. The residue was powdered and dried in vacuo in a rotary evaporator 60°C for 1 h to give the title compound as a white solid (25.0 g; 0.097 mol; 98% yield).

### Example 6: Preparation of crystalline 4-Amino-1-(2,3, 5-tri-O-acetyl-β-D-ribosyl)-s-triazin-2(1H)-one (Azacytidine Triacetate):

β-D-Ribofuranose tetracetate (34 g; 0.11 mol) was suspended in toluene and acetyl chloride (1.7 g; 0.02 mol) was added.

The mixture was stirred at 20-25°C and HCl gas (5.46 g; 0.15 mol) was bubbled over 8 h. The reaction mixture was concentrated under vacuum to residue (chloro-sugar).

A suspension of 5-azacytosine (10 g; 0.09 mol), hexamethyldisilazane (40 g; 0.13 mol), (NH4)₂SO₄ (0.2 g; 0.002 mol) and toluene (40 g) was heated to reflux (T= 108-114°C) for 8 h.

The reaction mixture was concentrated under vacuum to residue (silylazacitosine).

A solution of the chloro-sugar (0.15 mol) in MeCN was added into a mixture of sylil-azacytosine (0.11 mol) in MeCN and triflic (5.36 g; 0.036 mol) acid was added.

The resulting mixture was stirred at 20-25°C for 20-25 h, and then it was concentrated to residue. The latter was dissolved in CH₂Cl₂ (200 g) and the resulting solution was treated with a mixture of NaHCO₃ (7.5 g; 0.09 mol) and Na₂CO₃ (9.5 g; 0.09 mol) in H₂O (100 g) and vigorously stirred for 30 min.

The mixture was filtered on a dicalite cake, then the phases were separated and the aqueous layer was extracted with CH₂Cl₂.

The organic phases were collected, washed with water (2x25g) and concentrated under vacuum to an oily residue.

The oily residue was purified by chromatography (Silica gel; Eluent: EtOAc). The collected fraction were concentrated under vacuum to small volume and precipitated by addition of diisopropyl ether (130 mL). The solid was filtered off, washed with diisopropyl ether (100 mL) and dried under vacuum at 40°C (15.3 g; 0.041 mol; 46% yield).

### Example 7: crystallization of 5-Azacytidine.

Procedure: crude 5-azacytidine (10 g; HPLC assay 78%; 0.036mol) was suspended in DMPU (70 mL) and the resulting mixture was heated to 50°C for 30 min. The mixture was cooled to 20-25°C, maintained at the same temperature for 1 hour and then filtered. The solid was washed with DMPU (10 mL). The wet sample is suspended in *i*-PrOH (100 mL) and heated to 65-75 °C for 1 h and then filtered. The solid is washed with hot *i*-PrOH (30 mL) and dried under vacuum at 60°C. 5-Azacytidine (6 g; 0.025 mol) was obtained as a white solid. Yield: 69 %.

### Example 8: calculation of Azacytidine by the HPLC method.

The relative retention time (rrt) of each peak is determined according to the relative retention time of 5-Azacytidine.

Sample Solution A Chromatogram obtained (Diluent A - DMSO) - Integration of impurities peaks with a rrt not less than 0.5

In the chromatogram of Sample Solution A, obtained with DMSO as diluent, the percentage value of known and unknown peaks is calculated by the automatic integration method (area percent) with the following criteria:
- Disregard any peak with a relative retention time less than 0.5 (below rrt 0.5 is where DMSO elutes).
Sample Solution B Chromatogram (Diluent B - DMPU) ― Integration of impurities peaks with a rrt less than 0.5

In the chromatogram of Sample Solutions B, obtained with DMPU as diluent, the percentage value of known and unknown peaks is calculated by the automatic integration method (area percent) with the following criteria:
- Integrate all the peaks with a relative retention time less than 0.5 and the peak due to 5-Azacytidine.

### Calculation:

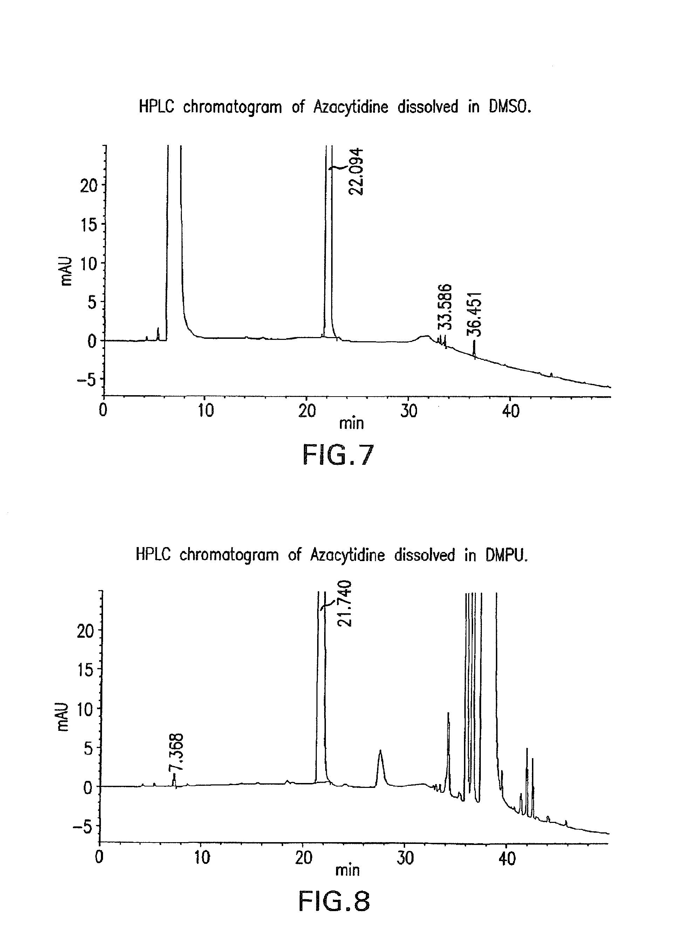

For DMSO: impurities having retention time of not less than 0.5, as depicted in Figure 7.
Impurity rrt 1.52=0.04%
Impurity rrt 1.64=0.07%

For DMPU: impurities having retention time of less than 0.5, as depicted in figure 8. Impurity rrt 0.34=0.09%

Purity of Azacytidine = 100-0.09-0.04-0.07= 99.80% area HPLC

Further aspects and embodiments of the invention are set out in the following clauses:
1. A process for preparing an intermediate of 4-amino-1-β-D-ribofuranosyl-1,3,5-triazin-2(1H)-one ("5-Azacytidine"), 4-amino-1-(2,3,5-tri-ester-β-D-ribosyl)-s-triazin-2(1H)-one, of the formula I: 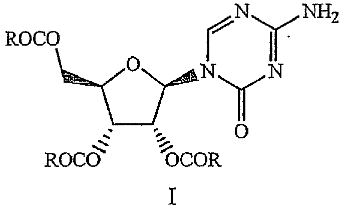 comprising reacting a silylated 5-azacytosine of the formula II, 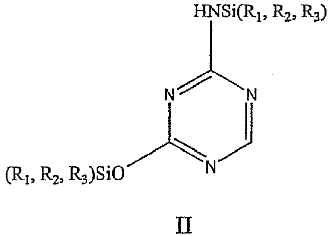 a sugar moiety having of the formula III: 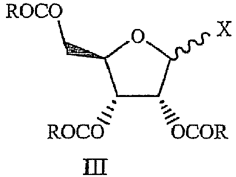 and a protic acid; wherein R is a substituted or non substituted C₁-C₂₀ acyl moiety, R₁, R₂ and R₃ are each independently H or an alkyl group, and X is a halogen.
2. The process of clause 1, wherein the C₁-C₂₀ acyl moiety is substituted with an aliphatic or branched alkyl, or with a benzyl group.
3. The process of clause 2, wherein the C₁-C₂₀ acyl moiety is C (O) CH₃ or C (O) phenyl
4. The process of any one of the preceding clauses, wherein the alkyl group in R₁, R₂ and R₃ in the compound of formula II is a C₁-C₄ alkyl group.
5. The process of any one of the preceding clauses, wherein the halogen is either Cl or Br.
6. The process of any one of the preceding clauses, wherein the 5-Azacytidine intermediate of formula I is prepared by combining a first mixture comprising the silylated 5-azacytosine of formula II, a second mixture comprising the sugar moiety of formula III and a protic acid to obtain a reaction mixture, comprising said intermediate of formula I.
7. The process of clause 6, wherein the protic acid is present in a catalytic amount.
8. The process of clause 7, wherein the protic acid is present in an amount of about 0.1 to about 0.9 mol/mol, in respect to the silylated 5-azacytosine of formula II.
9. The process of clause 8, wherein the protic acid is triflic acid.
10. The process of any one of clauses 8 and 9, wherein the first mixture comprises the silylated 5-azacytosine of formula II and an organic solvent.
11. The process of clause 10, wherein the organic solvent is selected from the group consisting of acetonitrile, methylene chloride and 1, 2- dichloromethane.
12. The process of any one of clauses8-11, wherein the second mixture is a solution of the sugar moiety of formula III in an organic solvent.
13. The process of clause 12, wherein the organic solvent is selected from the group consisting of acetonitrile, methylene chloride and 1, 2- dichloromethane, more preferably acetonitrile.
14. A process for preparing 4-amino-1-β-D-ribofuranosyl-1, 3, 5-triazin-2(1H)-one ("5-Azacytidine") of the formula IV: 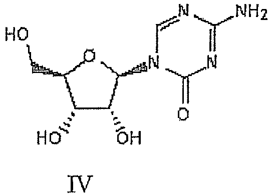 comprising preparing the intermediate of 5-Azacytidine of the formula I according to claim 1, and converting it to 4- amino-1-β-D-ribofuranosyl-1, 3, 5-triazin-2(1H)-one ("5-Azacytidine").
15. The process of clause14, wherein prior to converting the obtained intermediate 4-amino-1-(2, 3, 5-tri-ester-β-D-ribosyl)-s-triazin-2(1H)-one of formula I to 5-Azacytidine the reaction mixture containing it is concentrated to obtain a residue comprising the intermediate 4-amino-1-(2, 3, 5-tri-ester-β-D-ribosyl)-s-triazin-2(1H)-one of formula I, which reacts with a base.
16. The process of clause15, wherein optionally, prior to reacting the residue with the base, it is dissolved in a water-immiscible organic solvent, providing a solution which then reacts with an aqueous solution of the base.
17. The process of clause 16, wherein the water-immiscible organic solvent is a halogenated aliphatic hydrocarbon or ester.
18. The process of any one of clauses 16 and 17, wherein the base is an inorganic base.
19. The process of clause18, wherein the inorganic base is selected from the group consisting of NaHCO₃, Na₂CO₃, K₂CO₃, KHCO₃, NaOH and NH₄OH.
20. A method for determining the purity of 5-azacytidine comprising:
   a) providing a sample of 5-Azacytidine,
   b) dissolving a first part of the sample in DMSO, and a second part of the sample in DMPU, providing a first and second sample solution,
   c) injecting each sample solution onto an HPLC column under the same conditions, providing a first and second chromatogram, and
   d) determining the purity of Azacytidine and the amount of each impurity based on both chromatograms.

## Claims

1. A method for determining the purity of 5-Azacytidine comprising:
a) providing a sample of 5-Azacytidine,
b) dissolving a first part of the sample in dimethyl sulfoxide ("DMSO"), and a second part of the sample in dimethylpropyleneurea ("DMPU"), providing a first and a second sample solution,
c) injecting each sample solution onto an HPLC column under the same conditions, providing a first and a second chromatogram, and
d) determining the purity of 5-Azacytidine and the amount of each impurity based on both chromatograms.

2. The method of claim 1 wherein the concentration of each sample solution in step b) is determined prior to the injection onto the HPLC column.

3. The method of claim 1 wherein the concentration of each sample solution in step b) is of about 2.7 mg/ml to about 3.3 mg/ml.

4. The method of claim 3 wherein the concentration of each sample solution in step b) is about 3 mg/ml.

5. The method of claim 1 wherein determining the purity of 5-Azacytidine in step d is done by subtracting the total percentage of impurities from 100% and the total percentage of impurities is obtained as the sum of the percentage of each impurity (having relative retention time not less than 0.5) detected in the sample dissolved in DMSO and the percentage of each impurity (having relative retention time less than 0.5) detected in the sample dissolved in DMPU.
